# EUROPEAN PATENT APPLICATION

(11) **EP 4 018 935 A1**
(43) Date of publication of application: **29.06.2022**
(21) Application number: 20216877.9
(22) Date of filing: 23.12.2020
(51) Int. Cl.: A61B 8/00, A61B 8/08

(54) **METHOD FOR DETERMINING A GEOMETRY OF AN EAR CANAL OR A PORTION OF AN EAR OF A PERSON**

(71) Applicant: Sonova AG, 8712 Stäfa (CH)
(72) Inventor: KUIPERS, Erwin, 8712 Staefa (CH)
(74) Representative: Liedtke & Partner Patentanwälte

(57) **Abstract**

The invention relates to a method for determining a geometry of an ear canal or a portion of an ear of a person, the method comprising:
- filling the ear canal and/or the portion of the ear with a liquid or a gel,
- inserting a capacitive micromachined ultrasonic transducer probe or a piezoelectric micromachined ultrasonic transducer probe,
- acquiring data using the probe, processing the acquired data to obtain a 2D or 3D image of the ear canal and/or the portion of the ear.

## Description

### Technical Field

The invention relates to a method for determining a geometry of an ear canal or a portion of an ear of a person.

### Background of the Invention

When providing hearing impaired people with hearing aids it is desirable to use comfortable earpieces with adequate retention. This requires detailed knowledge of the ear canal and outer ear geometry. In addition, the location of the osseous / cartilaginous (also referred to as bony/cartilaginous junction) should be known. However, to identify the spatial zones in which overwaxing can be applied, knowledge of the local radial ear canal tissue compliance is desired.
Overwaxing is a term used for locally offsetting the earpiece wall radially, mostly for obtaining an acceptable wearing comfort and retention. Another reason for performing a certain level of overwaxing is to obtain a more tight acoustic sealing to enhance the hearing performance.

### Summary of the Invention

It is an object of the present invention to provide an improved method for determining a geometry of an ear canal or a portion of an ear of a person.

The object is achieved by a method according to claim 1.

Preferred embodiments of the invention are given in the dependent claims.

According to the invention a method for determining a geometry of an ear canal or a portion of an ear, e.g. a portion of an outer ear, of a person comprises:
- filling the ear canal and/or the portion of the ear with a liquid or a gel,
- inserting a capacitive micromachined ultrasonic transducer (CMUT) probe or a piezoelectric micromachined ultrasonic transducer (PMUT),
- acquiring data using the probe, processing the acquired data to obtain a 2D or 3D image of the ear canal and/or the portion of the ear. The measurement using a capacitive micromachined ultrasonic transducer probe is very fast (e.g. up to 40/100 MHz ultrasound frequency, the sampling rate can be very high). The measurement can be performed when the probe has been inserted and is located at an insertion depth or it can be performed also during the insertion process, while the probe is being moved. The patient will never be in perfect rest. Tissue movement due to varying blood flow strength and body movement due to respiration are some causes for this. It will be possible to determine the ear canal geometry based on ultrasound measurements using a correlation technique, even if the probe is in movement.

In an exemplary embodiment the ear canal is cleaned and/or inspected prior to filling in the liquid or gel.

In an exemplary embodiment the liquid or gel is tempered to a temperature of 15°C to 50 °C, in particular 28 °C to 38 °C, especially 28 °C to 35 °C, before being filled in.

In an exemplary embodiment, the person is positioned lying sideways before filling in the liquid or gel.

In an exemplary embodiment, during insertion, an actual insertion depth is monitored. This may be done by the probe or by one or more additional sensors.

In an exemplary embodiment, a warning is output when the probe reaches or falls below a predetermined distance to an eardrum of the ear. The design of the probe may be such, that touching the ear canal wall would not injure the skin. A soft material could be used as a bumper.

In an exemplary embodiment, at the desired insertion depth, the probe is fixated in position and/or orientation by at least one of a headband, a strap and a skin adhesive.

In an exemplary embodiment, processing the acquired data further includes determining mechanical properties of the wall of the ear canal and/or the underlying tissue. For example, mechanical properties may be determined by sending out ultrasonic waves and analyzing the reflected waves at different time instants. By changing the angle of the emitted waves, or by focusing the ultrasound on a point in the tissue, 3D spatial information can be obtained. Signal processing techniques like averaging, filtering, transform to the frequency domain may be applied. The mechanical properties may include visco-elastic properties, isotropic/anisotropic properties of the tissue and visco-plastic properties. Moreover, the mechanical properties determined may relate to a visco-elastic model.

In an exemplary embodiment, processing the acquired data further includes performing 3D mapping of osseous material surrounding the ear canal and determining a location of an osseous/cartilaginous junction. The change from tissue to osseous material is such that impinging ultrasonic waves will be reflected at the transition tissue-osseous material. By employing imaging techniques like array-based beamforming and -focusing, a 3D map can be generated by signal processing.

In an exemplary embodiment, processing the acquired data further includes performing a 3D elastography, generating a 3D map of elastic moduli of tissue surrounding the ear canal.

In an exemplary embodiment, processing the acquired data further includes performing a 3D mapping of at least one biometric parameter.

In an exemplary embodiment, the at least one biometric parameter is one of blood perfusion and fat content.

In an exemplary embodiment, processing the acquired data includes determining information about the length of the ear canal.

In an exemplary embodiment, the acquired and/or processed data are stored in a cloud. A cloud is understood to be a remote file hosting service designed to host user files.

According to an aspect of the present invention, a measurement device is provided, comprising a capacitive micromachined ultrasonic transducer probe or a piezoelectric micromachined ultrasonic transducer and a data processing unit configured to perform the data acquisition and processing according to the method as described above.

Employing the proposed method, multiple purposes can be served:
- 3D Scanning of the ear canal shape,
- Determination of the length and/or volume of the ear canal,
- Determination of the location of the bony/cartilaginous junction,
- Determination of mechanical tissue properties,
- Identification of outer ear diseases,
- Identification of general health related and biometric parameters.

The method may be employed for ear canal and tissue imaging in order to allow for an optimal fitting and -wearing comfort of an earpiece, e.g. for a hearing device such as a hearing aid or an ear bud or any other type of hearing instrument. Further scope of applicability of the present invention will become apparent from the detailed description given hereinafter. However, it should be understood that the detailed description and specific examples, while indicating preferred embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawing which is given by way of illustration only, and thus, is not limitive of the present invention, and wherein:

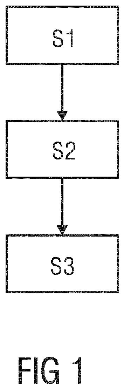

- Figure 1: is a schematic view illustrating an exemplary method for determining a geometry of an ear canal or a portion of the ear of a person.

### Detailed Description of Preferred Embodiments

**Figure 1** is a schematic view illustrating an exemplary method for determining a geometry of an ear canal or a portion of ear, in particular a portion of an outer ear, of a person.

The method for determining a geometry of an ear canal or a portion of the ear of a person procedure is proposed as follows:

An ear canal and outer ears of a person may be inspected according to common audiological working standards, including check for infections, cerumen, diseases, etc. If necessary, the ear canal and/or outer ear may be cleaned.

In a step S1, the ear canal and/or the portion of the ear, in particular the outer ear, be filled with a liquid or a gel for improving ultrasound transmission. The user may be positioned lying sideways for this purpose. In an exemplary embodiment, the liquid or gel is tempered to a temperature of 15 °C to 50 °C, in particular 28 °C to 38 °C, preferably 28 °C to 35 °C before being filled in.

In a step S2, a capacitive micromachined ultrasonic transducer probe or a piezoelectric micromachined ultrasonic transducer probe is inserted into the ear canal. During insertion, an actual insertion depth may be monitored, e.g. by the probe and/or using one or more additional sensors. A warning may be output when the probe reaches or falls below a predetermined distance to an eardrum of the ear. When the probe has reached the desired insertion depth, the probe may be fixated in position and/or orientation by at least one of a headband, a strap and a skin adhesive.

Once inserted and/or during insertion data may be acquired by the probe in a step S3, wherein the acquired data are processed to obtain a 2D or 3D image of the ear canal and/or the portion of the ear.

Processing data may further include one or more of:
- determining mechanical properties of the wall of the ear canal or the underlying tissue,
- performing 3D mapping of osseous material surrounding the ear canal and determining a location of an osseous/cartilaginous junction,
- performing a 3D elastography, generating a 3D map of elastic moduli of tissue surrounding the ear canal,
- performing a 3D mapping of at least one biometric parameter such as blood perfusion and fat content,
- determining information about the length and/or volume of the ear canal,
- performing a 3D mapping of the eardrum.

Mechanical properties may for example comprise the elastic modulus of the tissue, the tensile strength or hardness of the tissue. In order to determine such properties the invention proposes the use of a CMUT or PMUT. The ear canal, or the part of the ear under investigation is exposed to an ultrasound signal or a pulse signal. This signal travels to the wall of the ear canal and exerts a mechanical force on the surface and the underlying tissue. The reflected signal is detected by the CMUT or PMUT which acts as a hydrophone array. In medical imaging CMUT's have been used for examination of blood vessels. Since the ear canal is normally not filled with a liquid or gel CMUT's have not been used for this purpose. By measuring the time-delay between the ultrasound or pulse signal and the signal as picked up by the CMUT array the geometry of the ear canal can be determined. The time delay between the generation of the pulse and the arrival of the response in the single transducers of the CMUT array is proportional to the length the pulse has traveled through the liquid or gel. This allows reconstructing an image of the ear canal.

A reflected signal is dampened if the reflected surface is soft. From the intensity of the reflected signal the softness or dampening characteristic can be derived by a technician who is skilled in the art.

After the data acquisition the probe may be removed from the ear canal, the liquid or gel may be removed and the process may be repeated for the other ear of the person, if required.

The acquired and/or processed data may be stored in a secure way, e.g. they may be anonymized and stored in a cloud for cloud-based statistical analysis.

In an exemplary embodiment, processing of data may include forming a 3D elastic map (Young's modulus, shear modulus) by combining multiple slices.

In an exemplary embodiment the probe may be made of a more or less flexible material, where the transducer arrays are placed on a rigid section of the probe. For example, the probe may comprise a flexible printed circuit board or a flexible substrate which allows for folding or wrapping processes without damaging the electrical network.

In an exemplary embodiment the probe has a diameter of at most 6 millimeters.

In an exemplary embodiment the probe may have an elliptic or circular cross-sectional shape.

In an exemplary embodiment the probe has a smooth outer surface to not harm the patient.

In an exemplary embodiment, the probe may be equipped with a transducer array at its medial end to facilitate monitoring the distance to the eardrum.

In an exemplary embodiment, the probe may comprise an optical waveguide or a miniature camera for visual inspection purposes during the insertion process.

### List of References

- S1, S2, S3: step

## Claims

1. A method for determining a geometry of an ear canal or a portion of an ear of a person, the method comprising:
- filling the ear canal and/or the portion of the ear with a liquid or a gel,
- inserting a capacitive micromachined ultrasonic transducer probe or a piezoelectric micromachined ultrasonic transducer probe,
- acquiring data using the probe,
- processing the acquired data to obtain a 2D or 3D image of the ear canal and/or the portion of the ear.

2. The method of claim 1, wherein the ear canal is cleaned and/or inspected prior to filling in the liquid or gel.

3. The method according to claim 1 or 2, wherein the liquid or gel is tempered to a temperature of 15 °C to 50 °C, in particular 28 °C to 38 °C before being filled in.

4. The method according to any one of the preceding claims, wherein the person is positioned lying sideways.

5. The method according to any one of the preceding claims, wherein during insertion, an actual insertion depth is monitored.

6. The method of claim 5, wherein a warning is output when the probe reaches or falls below a predetermined distance to an eardrum of the ear.

7. The method according to any one of the preceding claims, wherein at the desired insertion depth, the probe is fixated in position and/or orientation by at least one of a headband, a strap and a skin adhesive.

8. The method according to any one of the preceding claims, wherein processing the acquired data further includes determining mechanical properties of the wall of the ear canal or the underlying tissue.

9. The method according to any one of the preceding claims, wherein processing the acquired data further includes performing 3D mapping of osseous material surrounding the ear canal and determining a location of an osseous/cartilaginous junction.

10. The method according to any one of the preceding claims, wherein processing the acquired data further includes performing a 3D elastography, generating a 3D map of elastic moduli of tissue surrounding the ear canal.

11. The method according to any one of the preceding claims, wherein processing the acquired data further includes performing a 3D mapping of at least one biometric parameter.

12. The method according to claim 11, wherein the at least one biometric parameter is one of blood perfusion and fat content.

13. The method according to any one of the preceding claims, wherein processing the acquired data includes determining information about the length and/or volume of the ear canal and/or performing a 3D mapping of an eardrum.

14. The method according to any one of the preceding claims, wherein the acquired and/or processed data are stored in a cloud.

15. A measurement device comprising a capacitive micromachined ultrasonic transducer probe or a piezoelectric micromachined ultrasonic transducer probe and a data processing unit configured to perform the data acquisition and processing according to the method of any one of the preceding claims.

## Amended claims

### Amended claims in accordance with Rule 137(2) EPC.

1. A method for determining a geometry of an ear canal or a portion of an ear of a person, the method comprising:
- filling the ear canal and/or the portion of the ear with a liquid or a gel,
- inserting a probe,
- acquiring data using the probe,
- processing the acquired data to obtain a 2D or 3D image of the ear canal and/or the portion of the ear,
**characterized in that** the probe is a capacitive micromachined ultrasonic transducer probe or a piezoelectric micromachined ultrasonic transducer probe, wherein the data are acquired during the insertion process, while the probe is being moved.

2. The method of claim 1, wherein the ear canal is cleaned and/or inspected prior to filling in the liquid or gel.

3. The method according to claim 1 or 2, wherein the liquid or gel is tempered to a temperature of 15 °C to 50 °C, in particular 28 °C to 38 °C before being filled in.

4. The method according to any one of the preceding claims, wherein the person is positioned lying sideways.

5. The method according to any one of the preceding claims, wherein during insertion, an actual insertion depth is monitored.

6. The method of claim 5, wherein a warning is output when the probe reaches or falls below a predetermined distance to an eardrum of the ear.

7. The method according to any one of the preceding claims, wherein at the desired insertion depth, the probe is fixated in position and/or orientation by at least one of a headband, a strap and a skin adhesive.

8. The method according to any one of the preceding claims, wherein processing the acquired data further includes determining mechanical properties of the wall of the ear canal or the underlying tissue.

9. The method according to any one of the preceding claims, wherein processing the acquired data further includes performing 3D mapping of osseous material surrounding the ear canal and determining a location of an osseous/cartilaginous junction.

10. The method according to any one of the preceding claims, wherein processing the acquired data further includes performing a 3D elastography, generating a 3D map of elastic moduli of tissue surrounding the ear canal.

11. The method according to any one of the preceding claims, wherein processing the acquired data further includes performing a 3D mapping of at least one biometric parameter.

12. The method according to claim 11, wherein the at least one biometric parameter is one of blood perfusion and fat content.

13. The method according to any one of the preceding claims, wherein processing the acquired data includes determining information about the length and/or volume of the ear canal and/or performing a 3D mapping of an eardrum.

14. The method according to any one of the preceding claims, wherein the acquired and/or processed data are stored in a cloud.

15. A measurement device comprising a capacitive micromachined ultrasonic transducer probe or a piezoelectric micromachined ultrasonic transducer probe and a data processing unit configured to perform the data acquisition and processing according to the method of any one of the preceding claims.
